# EUROPÄISCHE PATENTSCHRIFT

(11) **EP 1 605 260 B1**
(45) Veröffentlichungstag und Bekanntmachung des Hinweises auf die Patenterteilung: **16.07.2014**
(21) Anmeldenummer: 05104443.6
(22) Anmeldetag: 19.05.2005
(51) Int. Cl.: G01N 33/18, G01N 31/22, G01N 21/78

(54) **Verfahren und Testkit zur Bestimmung des organisch gebundenen Kohlenstoffs (TOC)**
Method and test kit for determining total organic carbon (TOC)
Procédé et dispositif pour determiner la teneur totale en carbone organique (TOC)

(30) Priorität: 09.06.2004 DE 102004028270; 09.08.2004 DE 102004038607
(43) Veröffentlichungstag der Anmeldung: 14.12.2005
(73) Patentinhaber: Hach Lange GmbH, 14163 Berlin (DE)
(72) Erfinder: Rieger, Claudia, 40227 Düsseldorf (DE); Schweden, Ulrike, 40882 Ratingen (DE); Lenhard, Markus, 41751 Viersen (DE); Grabert, Elmar, 44625 Herne (DE); Lundgreen, Ulrich, 40479 Düsseldorf (DE); Farjam, Aria, 6291 HD Vaals (NL)
(74) Vertreter: Patentanwälte ter Smitten Eberlein Rütten Partnerschaftsgesellschaft

(56) Entgegenhaltungen:
- EP-A- 0 663 239
- WO-A-00/75653
- DE-A1- 2 458 143
- DE-A1- 10 018 784
- US-A- 3 930 798
- US-B1- 6 180 413

## Beschreibung

Die vorliegende Erfindung betrifft ein Verfahren zur Bestimmung des organisch gebundenen Kohlenstoffs (TOC).

In der DE 19616760 A1 sind ein Verfahren und eine Vorrichtung zur kontinuierlichen Bestimmung des TOC-Wertes offenbart. Bei dem Verfahren wird die Probenlösung kontinuierlich in einen Mikroreaktor gepumpt, im Mikroreaktor auf eine vorgegebene Temperatur erhitzt und die organische Verbindung in der Probenlösung unter definiert erhöhter Temperatur und definiert erhöhtem Druck kontinuierlich oxidativ umgesetzt. Anschließend werden die gasförmigen Oxidationsprodukte aus der Probe durch eine Membran abgesaugt und in eine Messzelle geleitet und dort mittels eines Massenspektrometers gemessen.

In der WO 99/42824 A1 ist ein Verfahren zur Bestimmung des TOC-Gehaltes in Flüssigkeiten dargestellt, bei dem die Probenlösung in eine Reaktionskammer zur Oxidation des Kohlenstoffs eingeleitet wird und durch von außen in die Reaktionskammer eintretende Flüssigkeit in eine an die Reaktionskammer angeschlossene Messzelle überführt wird. Dort wird die Kohlendioxidkonzentration im Zuge des Durchströmens der Messzelle dynamisch erfaßt, wobei ein zur unbehandelten Flüssigkeit korrespondierender Grundwert der Kohlendioxidkonzentration sowie ein sich im Zuge des Durchflusses der Messzelle durch die mit Kohlendioxid angereicherte Flüssigkeit einstellender Maximalwert der Kohlendioxidkonzentration gemessen und wobei dann eine Differenz aus Maximalwert und Grundwert gebildet wird. Dabei wird die Kohlendioxidkonzentration anhand von Leitfähigkeitsmessungen ermittelt.

Zur Bestimmung des Analysenparameters TOC (Total Organic Carbon) in Wasserproben existieren seit einigen Jahren Testkits. So sind beispielsweise Testkits entwickelt worden, wie sie in der EP 0 663 239 B1 beschrieben sind. Mit dem dort beschriebenen System kann die Analytik einfach und schnell vor Ort durch wenig vorgebildetes Personal und mit preiswerten Mitteln durchgeführt werden. Der Testkit hat zwei als Küvetten ausgebildete Behälter, nämlich einen Probenaufnahmebehälter und einen Analysenbehälter, die jeweils obenseitig Behälteröffnungen aufweisen, die mit aufschraubbaren Verschlußkappen verschließbar sind. Zu dem Testkit gehört auch ein Adapter, über den die Behälteröffnungen nach Abnahme der Verschlüsse gasdicht miteinander verbindbar sind. Der Adapter ist mit einer semipermeablen Membran versehen, die für Gase und hier insbesondere den zu analysierenden Inhaltsstoff und das Trägergas durchlässig ist. Hierzu kann sie beispielsweise aus hydrophobem Material bestehen. Der Analysenbehälter kann das Indikatorreagenz in vorkonfektionierter und lagerfähiger Form enthalten. Ebenso kann auch der Probenaufnahmebehälter schon vorkonfektioniert mit einem Aufschlußreagenz versehen sein, der die Überführung des zu analysierenden Inhaltsstoffes in die Gasform bewirkt.

In der DE 10018784 C2 ist eine Abwandlung dieses Testkits beschrieben. Dabei weist der Analysenbehälter eine Druckentlastungseinrichtung auf, die vorzugsweise an dem der Behälteröffnung gegenüberliegenden Ende des Analysenbehälters angeordnet ist. Überschüssiges Trägergas entweicht durch die Druckentlastungseinrichtung, die ausschließlich für Gase durchlässig ist, vor allem wenn als Indikatorreagenz eine Flüssigkeit zur Anwendung kommt.

In der WO 00/75653 wird eine Analysevorrichtung beschrieben, die aus zwei ineinanderfügbaren Gefäßen besteht. Hierbei enthält das innere Gefäß den Indikator. Die zu analysierende Probe befindet sich in dem äußeren Gefäß. Beide Gefäße sind nur über den Gasraum miteinander verbunden. Durch Erhitzen werden die flüchtigen Substanzen aus der Probe in die Gasphase befördert und kommen über den Gasraum mit dem Indikator in Kontakt und erzeugen darin eine Änderung. Die Änderung des Indikators wird mittels der Transmission eines Lichtstrahls bestimmt.

In der DE 10121999 A1 ist ein Verfahren zur photometrischen oder fluorimetrischen Bestimmung von flüchtigen Substanzen in Lösungen beschrieben. Hierfür wird ein System eingesetzt, welches eine Küvette aufweist, welche durch eine ionenimpermeable, gaspermeable Membran in zwei Bereich aufgeteilt ist. Hierdurch weist die Küvette zwei getrennte Räume für Proben- und Aufschlußlösung einerseits und Indikatorlösung andererseits auf.

Aus der DE 2534620 A1 ist ein Verfahren zur Bestimmung des anorganischen Kohlenstoffgehalts von wässrigen Flüssigkeiten bekannt, bei dem eine Probe der zu analysierenden Flüssigkeit zusammen mit einem kohlendioxidfreien Trägergas in eine beheizte Reaktionskammer eingebracht wird, die anorganischen kohlenstoffhaltigen Verbindungen mit einem Reaktionsmittel zu Kohlendioxid zersetzt werden und das entstandene Kohlendioxid einen CO₂-Analysator zugeführt wird. Eine Bewegung der Reaktionskammer ist nicht erwähnt.

Um den TOC zu bestimmen, ist es regelmäßig erforderlich, den anorganischen Kohlenstoff zu entfernen. So wird beispielsweise in der DE 19906151 A1, der DE 19616760 A1, der DE 4307814 A1, der DE 10018784 C2, der DE 10121999 A1, der EP 0663239 B1 und der WO 00/75653 beschrieben, daß die anorganischen Kohlenstoffverbindungen durch Ansäuern und anschließendes Austreiben entfernt werden können.

Die beschriebenen, seit einigen Jahren existierenden Testkits weisen mithin folgenden typischen Arbeitsgang bei der Analysendurchführung auf:
1. Der anorganische Kohlenstoff der Probe (IC, Inorganic Carbon), bestehend aus physikalisch gelöstem Kohlendioxidgas und chemisch gebundenen Carbonaten, wird nach Ansäuern in Kohlendioxid umgewandelt und ausgetrieben,
2. die um den IC abgereicherte Probe wird in den Reaktionsbereich des TOC-Testkits gegeben,
3. ein chemisches Oxidationsmittel wird hinzugegeben,
4. der Reaktionsbereich wird über den Gasraum mit einer Indikatorlösung verbunden, welche ein für Kohlendioxid empfindliches Farbreagenz enthält,
5. der Reaktionsbereich wird erhitzt, wobei der chemisch gebundene organische Kohlenstoff durch das Oxidationsmittel in Kohlendioxid umgewandelt und dieses Gas in die Indikatorlösung übergetrieben wird,
6. der Reaktionsbereich wird abgekühlt,
7. die Farbveränderung der Indikatorlösung durch das übergetriebene Kohlendioxid wird in einem Photometer als Extinktion gemessen und aus der Extinktion mittels vorliegender Kalibrierdaten der TOC berechnet,
8. die benutzten Reaktionsbereiche (Küvetten) mit den verbrauchten Reagenzien werden in die Testkit-Packung zurückgegeben und später zur fachgerechten Entsorgung an den Lieferanten zurückgesendet.

Der erste Schritt, die Austreibung des IC, wird bei den bisher bekannten Verfahren in der Regel folgendermaßen durchgeführt:
1. Die Probe wird in einen Austreibbehälter, z.B. einen Erlenmeierkolben gegeben,
2. die Probe wird durch Hinzufügen einer Säure angesäuert,
3. ein Magnetrührstab wird in den Austreibbehälter mit der angesäuerten Proben gegeben,
4. die Probe wird auf einen Magnetrührer plaziert und 5 bis 10 Minuten gerührt (dabei wird der IC ausgetrieben),
5. mittels Pipette wird ein Aliquot der um den IC abgereicherten Probe in den Reaktionsraum des Küvettentest gegeben,
6. Austreibbehälter und Magnetrührstab werden gereinigt.

Dieses beschriebene Handling ist sehr aufwendig für den Anwender. Abgesehen davon bergen die notwendigen Reinigungsarbeiten an Austreibbehälter und Magnetrührer die Gefahr von Verunreinigungen und damit Verfälschungen der späteren Probenanalysen.

Aus der DE 4307814 A1 ist es für die Entfernung des anorganischen Kohlenstoffs bekannt, die zu untersuchende Probe mittels einer Säure, z.B. Salzsäure, auf einen schwachen pH-Wert von etwa 2 einzustellen und die Messmenge durch Einblasen eines Gases, z.B. von Luft, zum Aufwallen zu bringen. Infolge der Reaktion zwischen den Karbonaten und der Säure entsteht Kohlendioxid. Das Ausgasen des Kohlendioxids wird dadurch erreicht, daß die Probe durch eine aufwärtsgerichtete, oben offenmündende Messleitung zum Überlaufen gebracht wird und das austretende Kohlendioxid einschließlich der zuvor eingeblasenen Gasmenge abgeleitet wird. Auch dieses Verfahren ist aufwendig. Abgesehen davon ist das Risiko einer Ungenauigkeit der Analyse damit verbunden, daß Wasser aus dem Behältnis ausgeleitet wird.

Aufgabe der vorliegenden Erfindung ist es demgemäß, ein Verfahren der Umwandlung und Austreibung des anorganisch gebundenen Kohlenstoffs aus der Probe deutlich zu vereinfachen und die Gefahren von Verunreinigungen zu beseitigen. Zugleich soll erreicht werden, daß Testkits zur Verfügung gestellt werden, bei denen im Reaktionsbereich bereits ein saures Reagenz und ein oxidierendes Reagenz vorkonfektioniert (vom Hersteller fertig abgepackt) ist und der Arbeitsschritt des Ansäuerns und der Oxidationsmittelzugabe zur Probe eliminiert ist. Außerdem soll nur noch mit einem Reaktionsbereich gearbeitet werden, das heißt, Austreiben des anorganischen Kohlenstoffs und/oder Oxidation des organisch gebundenen Kohlenstoffs soll in ein und demselben Reaktionsbehälter durchgeführt werden. Hiermit wird auch das Überführen der Probe in die Reaktionsküvette vermieden. Ferner soll der Einsatz eines Magnetrührstabes vermieden werden. Zusammenfassend heißt dies, Aufgabe der vorliegenden Erfindung ist es, ein Minimalsystem zur Verfügung zu stellen, bei dem die Austreibung des anorganischen Kohlenstoffs im Reaktionsbereich erfolgen soll. Hierbei sollen mehrere Analysen parallel ausgetrieben werden.

Diese Aufgabe wird durch ein Verfahren gemäß Anspruch 1 sowie ein Testkit gemäß Anspruch 10 gelöst.

Das Entfernen des anorganischen Kohlenstoffs wird durch eine Säure ermöglicht.
Als Säuren werden vorzugsweise Phosphorsäure und Schwefelsäure, bzw. davon abgeleitete Puffer verwendet. Ganz besonders bevorzugt ist Phosphorsäure bzw., die hiervon abgeleiteten Puffer.

Durch Bewegen der Probe im Reaktionsbereich können Vorgänge beschleunigt werden, die dazu beitragen, daß das Kohlendioxid schnell aus dem Reaktionsbereich mit der angesäuerten Probe in die Umgebung abgegeben werden. Das sind im Wesentlichen hintereinander geschaltete Gleichgewichtsvorgänge. Hierzu gehören:
- Das Durchmischen der Flüssigen Probe, um immer wieder neues Kohlendioxid aus den Tiefen der Lösung an die flüssig-gasförmige Grenzfläche zu transportieren.
- Das Durchmischen des Gasraumes im Reaktionsbereich, um immer wieder neues Kohlendioxid aus den Tiefen des Gasraums an die Öffnung des Reaktionsbereichs zu transportieren.
- Der Abtransport des an der Öffnung austretenden Kohlendioxids in den Umgebungsgasraum.
   Erfindungsgemäß wird der Reaktionsbereich bzw. das Reaktionsgefäß horizontal und kreisförmig bewegt. Hierbei hat sich als vorteilhaft erwiesen, dass diese Bewegung eine Fliehkraftkomponente aufweist, so dass die Probe an die Wandungen des Reaktionsbereichs gepresst wird und zusammen mit der unten aufgeführten Strukturierung des Reaktionsbereichs (Schulter) ein quantitativer Verbleib im Reaktionsbereich gewährleistet wird. Auch können Schikanen und Strukturen an der Innenwandung des Reaktionsbereich angebracht werden, über welche die darüber hinweggleitende Probe zusätzlich gemischt wird.

Erfindungsgemäß liegt der Radius der Kreisbahn zwischen 0,5 und 5 mm. Bevorzugt ist ein Radius von 2 mm.

Die Kreisfrequenz liegt zwischen 1 und 1000 Hz, bevorzugt zwischen 1 und 100 Hz. Besonders bevorzugt sind 30 Hz.

Neben der Bewegung des Reaktionsbereichs , welche die oben aufgeführten Misch-und Transportvorgänge beschleunigen wird durch weitere Maßnahmen das Herausspritzen der Probe aus dem heftig bewegten Reaktionsbehälter verhindert.
Hierzu wird ein Reaktionsbereich bzw. -gefäß eingesetzt, welches unterhalb seiner Öffnung eine Wölbung in Form einer Schulter aufweist. D.h., die Küvette ist in ihrem Durchmesser vom Boden bis zur Öffnung nicht gleich. Vielmehr ist zur Öffnung hin der Durchmesser des Gefäßes verengt. Es hat sich erfindungsgemäß gezeigt, daß sich durch die Anordnung dieser Schulter ein Herausspritzen der Probe aufgrund der Bewegung vollständig vermeiden läßt.
Hier kann durch Veränderung der Formgebung der Schulter (z. B. schärferes Auspräge, zusätzliche Rückhaltenase) der Rückhalteeffekt erhöht, die mechanische Bewegung verstärkt und somit letztlich die Austreibzeit verkürzt werden. Darüber hinaus können Strukturen innerhalb des Schulterbereichs angebracht werden, die ähnlich einem Ableitblech, die herausstrebende Probe in den Reaktionsbereich zurückleiten. Selbstverständlich ist es auch möglich, den Verschluss des Reaktionsbereich mit Strukturen (z. B. einer Schulter) zu versehen.

Infolge der Ausgestaltung mit Schulter wird ein Betrieb des Reaktionsbereichs in vertikal stehender Form ermöglicht.

In einer besonders erfindungsgemäß bevorzugten Variante wird der Reaktionsbereich in eine Vorrichtung eingesetzt, welche ein oder mehrere Reaktionsbereiche aufzunehmen vermag. Diese Vorrichtung führt die beschriebenen Bewegungen durch. Durch Einsatz der beschriebenen Vorrichtung für mehrere Reaktionsbereiche wird die Möglichkeit einer parallelen Vorbereitung mehrerer Proben für die Analyse ermöglicht. Bei den herkömmlichen Methoden war die Vorbereitung der Probe unter anderem auch deshalb zeitaufwendig, weil ein Teil der typischen Anwender regelmäßig nur über einen einzigen Magnetrührer verfügt. Der gleichzeitige Ansatz bzw. die gleichzeitige Vorbereitung mehrerer Proben für die Analyse war mithin nicht möglich. D.h., bei den bisher bekannten Verfahren zur Probenvorbereitung mittels Magnetrührer und Erlenmeierkolben konnte eine Probenmehrzahl nur sequentiell und damit wesentlich zeitaufwendiger vorbereitet werden.

Das beschriebene erfindungsgemäße Ansäuern der Probe zur Umwandlung des anorganischen Kohlenstoffs in Kohlendioxid ermöglicht nunmehr ein wesentlich einfacheres Handhaben von Proben. Erfindungsgemäß wird ermöglicht, daß im Reaktionsbereich eine saure Lösung bereits vorkonfektioniert vom Hersteller fertig abgepackt geliefert wird. Der Arbeitsschritt des Ansäuerns in einem gesonderten Gefäß, z.B. in einem Erlenmeierkolben mit einem Magnetrührer entfällt vollständig. Das personal- und zeitaufwendige Reinigen und Spülen der Erlenmeierkolben entfällt ebenfalls. Erfindungsgemäß wird Ansäuern und anschließende Umwandlung des anorganischen Kohlenstoffs sowie daran anschließende Umwandlung des organisch gebundenen Kohlenstoffs in Kohlendioxid in ein- und demselben Reaktionsbereich durchgeführt. Die typischen Probleme, welche mit einer ungenügenden Reinigung der Gefäße (d.h., der eingesetzten Erlenmeierkolben bei der Ansäuerung) werden somit erfindungsgemäß vermieden.

Gegenstand der Erfindung ist somit auch ein Testkit zur Bestimmung des organisch gebundenen Kohlenstoffs (TOC) gemäß Anspruch 10, welches wenigstens ein Reaktionsgefäß bzw. einen Reaktionsbereich umfaßt, wobei der Reaktionsbereich Stoffe zur Erzeugung von Kohlendioxid aus der Probe in vorkonfektionierter und lagerfähiger Form und der Detektionsbereich wenigstens ein gassensitives Reagenz in fester oder flüssiger sowie vorkonfektionierter und lagerfähiger Form enthält. Der Reaktionsbereich zeichnet sich hierbei dadurch aus, daß er in vorkonfektionierter Form Stoffe, bevorzugt Säuren zur Umwandlung des anorganischen Kohlenstoffs in Kohlendioxid und darüber hinaus auch Stoffe zur Umwandlung (Oxidationsmittel) des in der Probe enthaltenen TOC zum gasförmigen Kohlendioxid für den später durchzuführenden Schritt d) in vorkonfektionierter und lagerfähiger Form enthält. Hierbei können besondere Maßnahmen getroffen werden um zu verhindern, daß die anwesenden Oxidationsmittel während des Austreibens des anorganischen Kohlenstoffs in Schritt b) schon TOC in Kohlendioxid umwandeln:
- feste Oxidationsmittel zusetzen, die sich in der Kälte nicht oder schlecht lösen
- Oberfläche der festen Oxidationsmittel durch Kompaktierung (z. B. Tablettierung) reduzieren
- Oxidationsmittel einsetzen, die in der Kälte kein TOC oxidieren

In einer weiteren Variante des erfindungsgemäßen Verfahrens wird über die Öffnung des Reaktionsbereichs bzw. Reaktionsgefäßes ein Luftstrom geleitet. Hierdurch wird gewissermaßen eine Sogwirkung erzeugt, so daß das Austreiben des Kohlendioxids aus der Öffnung des Reaktionsbereichs bzw. Reaktionsgefäßes beschleunigt wird. Vorzugsweise wird hierfür eine vorgereinigte Umgebungsluft eingesetzt.

Die Luftbewegung kann im einfachsten Falle durch Anordnung eines Ventilators erreicht werden. Ebenso ist es aber auch möglich, z.B. mittels einer Düse einen gezielten Luftstrom über die Öffnung des Reaktionsbereichs bzw. Reaktionsgefäßes streichen zu lassen. Durch die damit verbundene höhere Geschwindigkeit des Luftstroms kann eine weitere Beschleunigung des Transport des Kohlendioxidgases aus dem Reaktionsbereich bzw. Reaktionsgefäß heraus erreicht werden.

Ebenso ist es aber auch möglich, durch einen pulsierenden Luftstrom eine Beschleunigung des Gasaustrags zu erreichen. Ein solches Pulsieren kann im einfachsten Falle dadurch erreicht werden, daß in Folge der Bewegungen des Reaktionsbereichs bzw. Reaktionsgefäßes eine ständige Umlenkung des Luftstroms erfolgt. Hierbei kann durch das Pulsieren ebenfalls die im Reaktionsbereich befindliche Probe pulsierend bewegt werden, wobei ein zusätzliches Mischen in der flüssigen Probe entsteht, was wiederum zu einem beschleunigten Austreiben führt.

Im folgenden wird die Erfindung unter Bezugnahme auf die Abbildungen näher beschrieben:

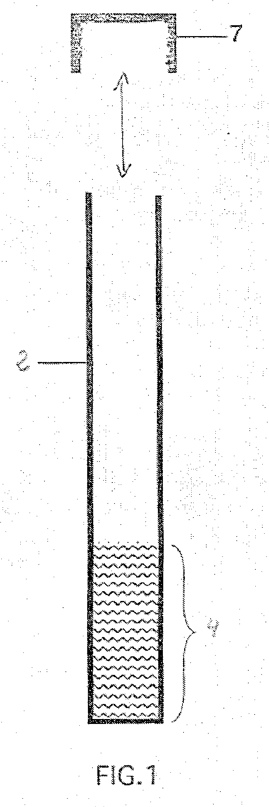

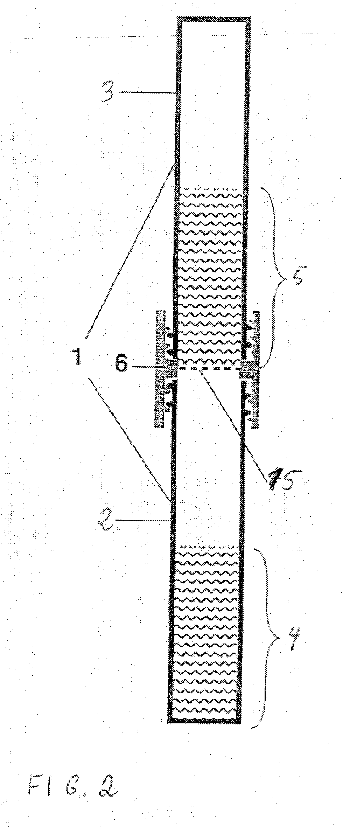

Figur 1 zeigt ein Beispiel für einen nicht erfindungsgemäßen Reaktionsbereich. Dieser Bereich besteht im wesentlichen aus einem verschließbaren Behälter mit einer Verschlußvorrichtung 7, vorzugsweise einer Schraubverschlußkappe. Nach Abnahme der Kappe 7 wird die Probe in den Reaktionsbereich 2 gegeben. Dieser Reaktionsbereich enthält vorzugsweise eine vorkonfektionierte Reaktionslösung 4. Diese Reaktionslösung kann zum einen Reagenzien zum Umwandeln des anorganisch gebundenen Kohlenstoffs enthalten und zum anderen auch Reagenzien zum Umwandeln des organisch gebundenen Kohlenstoffs. Ebenso ist es aber auch möglich, die Probe zunächst in den Reaktionsbereich 2 einzugeben und sodann eine vom Hersteller mitgelieferte Umwandlungslösung für den anorganischen Kohlenstoff einzusetzen. Hierbei handelt es sich in der Regel um Säuren. Nach Zusatz der Säuren und der Probe wird das freiwerdende Kohlendioxid mittels Bewegung ausgetrieben. Die weitere Behandlung bzw. Durchführung der Analyse kann in einer der Vorrichtungen gemäß den Figuren 2 bis 5 erfolgen.
Figur 2 zeigt ein Beispiel einer Vorrichtung, wie sie aus der EP 0 663 239 A2 bekannt ist. Hier wird, in Analogie zur Beschreibung zu Fig. 1, nach Zusatz der Säuren und der Probe in den Reaktionsbereich 2 das freiwerdende Kohlendioxid mittels Bewegung ausgetrieben. Danach wird der Reaktionsbereich 2 mit dem Detektionsbereich 3 verbunden. Im Reaktionsbereich 2 befindet sich die Reaktionslösung 4 und im Detektionsbereich 3 die Indikatorlösung 5. Die Verbindung der beiden Bereiche erfolgt über den Adapter 6, welcher eine Membran 15 aufweist. Für die Erzeugung der gasförmigen Bestandteile aus der Probe 4 können physikalische, chemische, biochemische oder mikrobiologische Methoden angewandt werden. Als chemische Methoden seien vorzugsweise Ansäuern, Alkalisieren, Oxidieren, Reduzieren und Derivatisieren genannt. Methoden zur Beschleunigung der Gasbildung sind beispielsweise in der EP 0 663 239 B1 beschrieben. Vorzugsweise wird eine Behandlung mit Oxidationsmittel, z.B. Persulfat vorgenommen und der Reaktionsbereich 2 erhitzt. Der chemisch gebundene organische Kohlenstoff wird durch das Oxidationsmittel in Kohlendioxid umgewandelt und dieses Gas in die Indikatorlösung 5 übergetrieben. Der Reaktionsbereich wird abgekühlt. Die Farbveränderung der Indikatorlösung durch das übergetriebene Kohlendioxid wird in einem Photometer als Extinktion gemessen. Aus der Extinktion wird mittels vorliegender Kalibrierdaten der TOC berechnet.

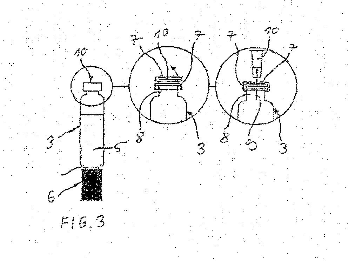

Die Figur 3 zeigt einen Testkit, wie er aus der DE 10018784 C2 bekannt ist. Im Unterschied zu dem Testkit gemäß Figur 2 ist hier eine Verbindung des Detektionsbereichs 3 mit der Außenatmosphäre vorgesehen. Das über den Adapter 6 in die Indikatorlösung 5 des Reaktionsgefäßes 2 eingetriebene Gas wird wie in dem Beispiel gemäß Figur 2 beschrieben, gemessen. Mittels der Nadel 10 kann der auf die Öffnung 8 aufgesetzte Verschluß 7 nach außen hin geöffnet werden, um so über die Kanüle 9 eine Druckentlastung zu erzeugen. Dies kann zu einer zusätzlichen Beschleunigung des Gastransports beitragen. Hier wird, in Analogie zur Beschreibung zu Fig. 1, nach Zusatz der Säuren und der Probe in den Reaktionsbereich, der analog zu Fig. 2 ausgeprägt ist, das freiwerdende Kohlendioxid ausgetrieben.

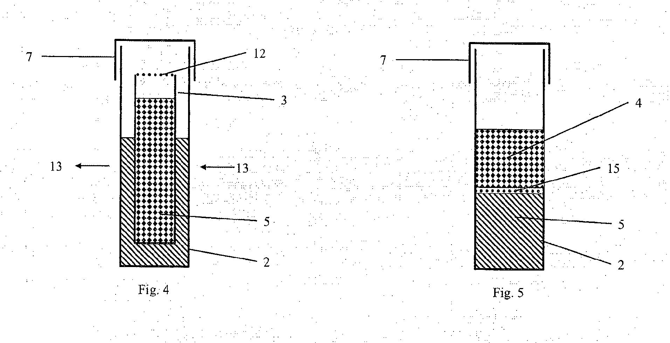

Figur 4 zeigt ferner eine Vorrichtung, wie sie aus der WO 00/75653 bekannt ist. In diesem Beispiel ist in den Reaktionsbereich 2 der Detektionsbereich 3 mit der Indikatorlösung 5 eingesetzt. Über den Gasraum sind die Gefäße miteinander verbunden. Hier wird, in Analogie zur Beschreibung zu Fig. 1, nach Zusatz der Säuren und der Probe in den Reaktionsbereich 2 das freiwerdende Kohlendioxid mittels Bewegung ausgetrieben. Durch Erhitzen wird aus den gebundenen organischen Kohlenstoffen erzeugten Kohlendioxid aus der Probe befördert und mit dem Indikator 5 in Kontakt gebracht. Die Änderung des Indikators wird mittels der Transmission eines Lichtstrahls 13 gemessen. In einer besonderen Ausführungsform können Reaktions- und Detektionsbereich über eine Membran 12 miteinander verbunden sein.
Figur 5 zeigt ferner ein Beispiel für einen Testkit, wie er aus der DE 10121999 A1 bekannt ist. Die Indikatorlösung 5 ist durch eine Membran 15 von der Reaktionslösung 4 getrennt. Über der Reaktionslösung 4 befindet sich ein Gasraum 14 und die Verschlußkappe 7. Zur Bestimmung des TOC wird die Küvette mit ihrem Verschlußdeckel 7 geöffnet und in Analogie zur Beschreibung zu Fig. 1, nach Zusatz der Säuren und der Probe in den Reaktionsbereich 2 das freiwerdende Kohlendioxid mittels Bewegung ausgetrieben. Nach Verschließen mit dem Deckel 7 wird die Küvette umgedreht und in einen Thermostaten eingesetzt, wo durch Erhitzen des Reaktionsbereichs 4 aus dem gebundenen organischen Kohlenstoff Kohlendioxid erzeugt wird, welches aus der Probe befördert und mit dem Indikator 5 in Kontakt gebracht wird. Im Anschluß daran erfolgt eine Messung der Farbänderung des Indikators 5.

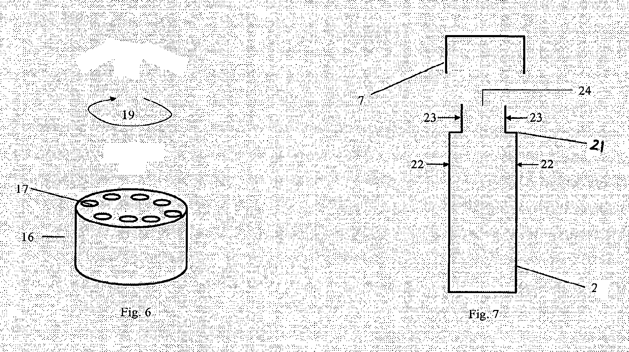

Figur 6 zeigt eine Variante für den Einsatz in dem erfindungsgemäßen Verfahren. Neben dem Ansäuern der Probe zur Entfernung des anorganischen Kohlenstoffs ist als zusätzliche Maßnahme eine Bewegung des Reaktionsbereich 2 vorgesehen. Diese Bewegungen erfolgen erfindungsgemäß in Form horizontalem von Kreisbewegungen 19. In der Variante gemäß Figur 6 kann zu diesem Zweck der Reaktionsbereich in die Vorrichtung 16 eingesetzt werden. In dieser Vorrichtung sind vorzugsweise mehrere Öffnungen 17 vorgesehen. Hierdurch wird erreicht, daß eine Vielzahl von Reaktionsbereichen zugleich in die Vorrichtung eingesetzt werden können. Durch die Bewegung erfolgt eine Durchmischung der Reaktionslösung mit dem Umwandlungsmittel, wofür vorzugsweise Säuren verwendet werden.
Figur 7 zeigt eine erfindungsgemäße Ausführungsform des Reaktionsgefaßes 2. Dieser Bereich weist ebenfalls eine Verschlußvorrichtung 7 auf. Im Unterschied zu der Vorrichtung gemäß Figur 1 ist das Reaktionsgefaß 2 mit einer Schulter 21 ausgerüstet. Hierdurch wird eine Verengung des Durchmessers 22 auf den Durchmesser 23 der Öffnung 24 erreicht. Bei Anwendung dieser Küvette läßt sich in Kombination mit der Vorrichtung gemäß Figur 6 eine optimale Bewegungsfrequenz erreichen. Reaktionslösung und Säuerungsmittel werden derart durchmischt, daß eine Beschleunigung des Austritts des aus dem anorganischen Kohlenstoffs entstandenen Kohlendioxids und des in der Probe vorhandenen gelösten Kohlendioxids erreicht wird, gleichzeitig wird ein Austritt aus dem Reaktionsgefaß vermieden.

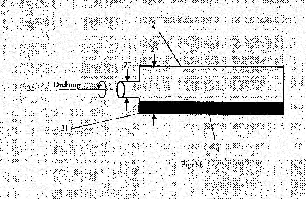

Figur 8 zeigt eine nicht erfindungsgemäße Ausführungsform des Reaktionsbereichs 2. Der Reaktionsbereich ist ebenfalls mit einer Schulter 21 ausgestattet. Durch die Durchmesserverengung kann der Reaktionsbereich liegend bewegt werden, ohne dass Reaktionslösung und/oder Probe 4 austritt. Hierbei sind alle vorher beschriebenen Bewegungen möglich. Bevorzugt ist eine Bewegung um die Drehachse 25. Hierbei bildet sich ein Flüssigkeitsfilm auf der Innenwandung des Reaktionsbereichs, welcher einen beschleunigten Gasaustausch hervorruft.

## Patentansprüche

1. Verfahren zur Bestimmung des organisch gebundenen Kohlenstoffs (TOC) in einer Vorrichtung (1), welche wenigstens ein Reaktionsgefäß (2) mit einer Öffnung (24) und einer Wölbung (Schulter) (21) unterhalb der Öffnung (24) und wenigstens ein Detektionsgefäß (3) aufweist, wobei:
a) die Probe in das Reaktionsgefäß (2) der Vorrichtung (1) gegeben wird,
b) der anorganische Kohlenstoff ausgetrieben wird,
c) die Vorrichtung (1) verschlossen wird,
d) mittels physikalischer, chemischer, biochemischer oder mikrobiologischer Methoden der organisch gebundene Kohlenstoff in gasförmiges Kohlendioxid umgewandelt,
e) das gasförmige Kohlendioxid in das Detektionsgefäß (3) überführt wird, und
f) anhand der Farbveränderung des Indikators der Kohlendioxidgehalt mit an sich bekannten Messverfahren bestimmt wird,
**dadurch gekennzeichnet, dass** in Schritt b) zur Austreibung des durch Umwandlung des anorganischen Kohlenstoffs entstandenen Kohlendioxids das vertikal stehende Reaktionsgefäß (2) horizontal und kreisförmig bewegt wird, wobei der Radius der Kreisbahn des Reaktionsgefäßes (2) zwischen 0,5 und 5 mm und die Kreisfrequenz des Reaktionsgefäßes (2) zwischen 1 und 1000 Hz liegt, so dass der anorganische Kohlenstoff in weniger als 5 min ausgetrieben wird.

2. Verfahren nach Anspruch 1, **dadurch gekennzeichnet, dass** in Schritt b) durch Zugabe von Säure(n) der anorganische Kohlenstoff in Kohlendioxid umgewandelt wird.

3. Verfahren nach Anspruch 2,
**dadurch gekennzeichnet, dass** als Säure(n) zur Umwandlung des anorganischen Kohlenstoffs Phosphorsäure, Schwefelsäure und davon abgeleitete Puffer im Reaktionsgefäß (2) eingesetzt werden.

4. Verfahren nach Anspruch 3, **dadurch gekennzeichnet, dass** die Säuren im Reaktionsgefäß (2) fertig konfektioniert enthalten sind.

5. Verfahren nach einem der vorhergehenden Ansprüche,
**dadurch gekennzeichnet, dass** über der Öffnung des Reaktionsgefäßes (2) ein Luftstrom erzeugt wird, der einen Austausch des Gasraums innerhalb des Reaktionsgefäßes mit der Umgebungsluft erzeugt.

6. Verfahren nach Anspruch 5,
**dadurch gekennzeichnet, dass** die Umgebungsluft vorgereinigt wird.

7. Verfahren nach einem der Ansprüche 5 oder 6,
**dadurch gekennzeichnet, dass** die Luftbewegung mittels eines Ventilators erzeugt wird.

8. Verfahren nach einem der Ansprüche 5 bis 7,
**dadurch gekennzeichnet, dass** der Luftstrom in Form einer laminaren Strömung auf die Öffnung des Reaktionsgefäßes (2) gerichtet ist.

9. Verfahren nach einem der Ansprüche 5 bis 8,
**dadurch gekennzeichnet, dass** ein pulsierender Luftstrom erzeugt wird.

10. Testkit zur Durchführung eines Verfahrens nach einem der vorhergehenden Ansprüche, umfassend:
a) wenigstens ein Reaktionsgefäß (2) mit einer Öffnung (24) und einer Wölbung (Schulter) (21) unterhalb der Öffnung (24) und wenigstens ein Detektionsgefäß (3),
b) wenigstens ein Mittel zur Umwandlung des anorganischen Kohlenstoffs in Kohlendioxid,
c) wenigstens ein Mittel zur Umwandlung des gebundenen organischen Kohlenstoffs in Kohlendioxid, vorzugsweise ein Oxidationsmittel, und
d) wenigstens ein gassensitives Reagenz, welches bei Kontakt mit Kohlendioxid eine Farbänderung erfährt,
wobei die Mittel b) und c) in vorkonfektionierter und lagerfähiger Form im Reaktionsgefäß (2) sowie das Mittel d) in vorkonfektionierter und lagerfähiger Form im Detektionsgefäß (3) enthalten sind,
und wobei das Reaktionsgefäß (2) und seine Wölbung (Schulter) (21) so ausgebildet sind, dass bei einer horizontalen und kreisförmigen Bewegung des vertikal stehenden Reaktionsgefäßes (2) mit einem Kreisbahnradius zwischen 0,5 und 5 mm und einer Kreisfrequenz zwischen 1 und 1000 Hz der anorganische Kohlenstoff in weniger als 5 min ausgetrieben und dabei ein Herausspritzen der Probe vollständig vermieden wird.

11. Verwendung des Testkits nach Anspruch 10 zur Bestimmung des organisch gebundenen Kohlenstoffs (TOC).

## Claims

1. Method for determining the organically bound carbon (TOC) in an apparatus (1), which comprises at least one reaction vessel (2) with an opening (24) and a curvature (shoulder) (21) below the opening (24) and at least one detection vessel (3),
a) the sample being placed into the reaction vessel (2) of the apparatus (1),
b) the inorganic carbon being expelled,
c) the apparatus (1) being sealed,
d) the organically bound carbon being converted into gaseous carbon dioxide by means of physical, chemical, biochemical or microbiological methods,
e) the gaseous carbon dioxide being transferred to the detection vessel (3, 3a) and
f) the carbon dioxide content being determined by means of measurement methods known per se on the basis of the color change of the indicator,
**characterized in that**, in step b), to expel the carbon dioxide formed by conversion of the inorganic carbon, the vertically upright reaction vessel (2) is moved horizontally and circularly, the radius of the orbit of the reaction vessel (2) being between 0.5 and 5 mm and the angular frequency of the reaction vessel (2) being between 1 and 1000 Hz so that the inorganic carbon is expelled in less than 5 minutes.

2. Method according to claim 1, **characterized in that**, in step b), the inorganic carbon is converted into carbon dioxide by adding acid(s).

3. Method according to claim 2, **characterized in that**, as acid(s) for converting the inorganic carbon, use is made in the reaction vessel (2) of phosphoric acid, sulphuric acid and buffers derived therefrom.

4. Method according to claim 3, **characterized in that** the acids in the reaction vessel (2) are present in ready-to-use formulation.

5. Method according to one of the preceding claims, **characterized in that**, above the opening of the reaction vessel (2), an air stream is generated which produces an exchange of the gas volume within the reaction vessel with the ambient air.

6. Method according to claim 5, **characterized in that** the ambient air is prepurified.

7. Method according to one of claims 5 or 6, **characterized in that** the air movement is generated by means of a fan.

8. Method according to one of claims 5 to 7, **characterized in that** the air stream is directed in the form of a laminar flow against the opening of the reaction vessel (2).

9. Method according to one of claims 5 to 8, **characterized in that** a pulsed air stream is produced.

10. Test kit for carrying out a method according to one of the preceding claims, comprising:
a) at least one reaction vessel (2) with an opening (24) and a curvature (shoulder) (21) below the opening (24) and at least one detection vessel (3),
b) at least one agent for converting the inorganic carbon into carbon dioxide,
c) at least one agent for converting the bound organic carbon for the conversion into carbon dioxide, preferably an oxidizing agent, and
d) at least one gas-sensitive reagent which experiences a color change on contact with carbon dioxide,
wherein the agents b) and c) are present in the reaction vessel (2) in a preformulated and storable form and the agent d) is present in the detection vessel (3) in a preformulated and storable form,
and wherein the reaction vessel (2) and its curvature (shoulder) (21) are designed such that when the vertically upright reaction vessel (2) is moved horizontally and circularly, with the radius of the orbit of the reaction vessel (2) being between 0.5 and 5 mm and the angular frequency of the reaction vessel (2) being between 1 and 1000 Hz, the inorganic carbon is expelled in less than 5 minutes and a spurting of the sample is altogether avoided.

11. Use of the test kit according to one of claims 10 for determining the organically bound carbon (TOC).

## Revendications

1. Procédé pour déterminer la teneur totale en carbone organique (TOC) dans un appareil (1) comprenant au moins un récipient de réaction (2) ayant une ouverture (24) avec une courbure (épaule) (21) au-dessous de l'ouverture (24), et au moins un récipient de détection (3), dans lequel
a) l'échantillon est introduite dans le récipient de réaction (2) dudit appareil (1),
b) le carbone inorganique est expulsé,
c) l'appareil (1) est fermé,
d) le carbone organique est converti en dioxyde de carbone gazeux par l'utilisation de méthodes physiques, chimiques, biochimiques or microbiologiques,
e) le dioxyde de carbone gazeux est transféré dans le récipient de détection (3), et
f) la teneur en dioxyde de carbone est déterminée par le changement de couleur d'un indicateur, utilisant des procédés de mesure connus en soi,
**caractérisé en ce que** dans l'étape b), afin d'expulser le dioxyde de carbone formé par la conversion du carbone organique, le récipient de réaction (2) disposé verticalement est mu horizontalement et circulairement, le radius de trajectoire circulaire du récipient de réaction (2) étant entre 0,5 et 5 mm et la fréquence angulaire du récipient de réaction (2) étant entre 1 et 1000 Hz de sorte que le carbone inorganique est expulsé en moins de 5 min.

2. Procédé selon la revendication 1, **caractérisé en ce que** dans l'étape b), le carbone inorganique est converti en dioxyde de carbone par addition d' (des) acide(s).

3. Procédé selon la revendication 2, **caractérisé en ce que** l'on utilise dans le récipient de réaction (2) comme acide(s) pour la conversion du carbone inorganique l'acide phosphorique, l'acide sulfurique et des tampons dérivés de ceux-ci.

4. Procédé selon la revendication 3, **caractérisé en ce que** les acides sont contenus dans le récipient de réaction (2) de façon prêt à l'emploi.

5. Procédé selon l'une quelconque des revendications précédentes, **caractérisé en ce qu'**un courant d'air est généré au-dessus de l'ouverture du récipient de réaction (2), qui provoque un échange entre l'espace de gaz au sein du récipient de réaction (2) et l'air ambiant.

6. Procédé selon la revendication 5, **caractérisé en ce que** l'air ambiant est purifié à préalable.

7. Procédé selon l'une des revendications 5 ou 6, **caractérisé en ce que** le mouvement d'air est provoqué par un ventilateur.

8. Procédé selon l'une des revendications 5 à 7, **caractérisé en ce que** le courant d'air est dirigé vers l'ouverture du récipient de réaction (2) sous forme d'un courant laminaire.

9. Procédé selon l'une des revendications 5 à 8, **caractérisé en ce qu'**un courant d'air pulsé est généré.

10. Kit de test pour l'exécution d'un procédé selon l'une quelconque des revendications précédentes, comprenant:
a) au moins un récipient de réaction (2) avec une ouverture (24) et une courbure (épaule) (21) au-dessous de l'ouverture (24), et au moins un récipient de détection (3),
b) au moins un moyen pour convertir le carbone inorganique en dioxyde de carbone,
c) au moins un moyen, de préférence un oxydant, pour convertir le carbone organique en dioxyde de carbone, et
d) au moins un réactif sensible à gaz dont le couleur se change lors du contact avec le dioxyde de gaz,
les moyens b) et c) étant contenus dans le récipient de réaction (2) de façon prêt à l'emploi et stockable, ledit moyen d) étant contenu dans le récipient de détection (3) de façon prêt à l'emploi et stockable,
et ledit récipient de réaction (2) et sa courbure (épaule) (21) étant formée de façon que, pendant un mouvement horizontal et circulaire du récipient de réaction (2) disposé verticalement avec un radius de trajectoire circulaire entre 0,5 et 5 mm et une fréquence angulaire entre 1 et 1000 Hz, le carbone inorganique est expulsé en moins de 5 min et le giclement de l'échantillon est entièrement évité.

11. Utilisation du kit de test selon la revendication 10 pour la détermination de la teneur en carbone organique (TOC).
